# DEMANDE DE BREVET EUROPEEN

(11) **EP 0 927 550 A1**
(43) Date de publication de la demande: **07.07.1999**
(21) Numéro de dépôt: 98490038.1
(22) Date de dépôt: 29.12.1998
(51) Int. Cl.: A61F 13/58

(54) **Article d'hygiène notamment couche-culotte, munie de pattes d'attache à propriété mécanique et adhésive**

(30) Priorité: 30.12.1997 FR 9716857
(71) Demandeur: Proteco, 59494 Petite Forêt (FR)
(72) Inventeur: Brutin, Jean-Marc, 59780 Camphin en Pevele (FR)
(74) Mandataire: Matkowska, Franck

(57) **Abrégé**

Article d'hygiène (1), notamment couche-culotte, comprenant une feuille imperméable extérieure (2), une feuille perméable intérieure (3), un système d'attache mécanique auto-agrippant composé de deux pattes d'attache latérales (5), fixées supportant des premiers éléments mécaniques du type crochets (6). Chaque patte d'attache latérale (5) comprend une bande principale (7) dont l'extrémité proximale (7a) est fixée sur la feuille extérieure (2) et dont l'extrémité distale (7b) porte les premiers éléments d'attache mécaniques (6) et une bande secondaire adhésive (8) dont la face adhésivée (8) est fixée, selon son extrémité proximale (8a), sur la feuille perméable intérieure (3) et, selon son extrémité distale (8b), sur la bande principale (7) à proximité immédiate des premiers éléments d'attache mécaniques (6). La bande principale (7) comporte une zone non adhérente (9), en amont de l'extrémité distale (8b) de la bande secondaire (8), et la bande secondaire (8) présente un pli (10) immédiatement en amont de la zone non adhérente (9) et une ligne de prédécoupe (13) dans le creux (12) en amont dudit pli (10).

## Description

La présente invention concerne le domaine des articles d'hygiène, et en particulier des articles jetables du type couches-culottes. Elle concerne plus particulièrement un article de ce type qui est pourvu de pattes d'attache de fermeture à éléments d'attache auto-agrippants du type crochets qui coopèrent avec des éléments d'attache du type boucles portés par une bande frontale peur la fermeture de l'article sur le corps de l'usager en utilisation normale et qui est également pourvu d'un élément adhésif pour la fermeture de l'article sur lui-même après usage.

On a déjà proposé dans le document EP 321 232 un article d'hygiène muni de pattes d'attache qui combinent des propriétés mécaniques et des propriétés adhésives. Selon ce document lors de la fermeture de l'article autour du corps de l'usager, c'est-à-dire en cours d'utilisation normale, l'élément d'attache mécanique de la patte d'attache vient s'accoupler avec l'élément d'attache mécanique de la bande frontale et le moyen de fixation adhésif est lui-même fixé soit sur la bande frontale soit sur une partie du corps de l'article d'hygiène et notamment la feuille extérieure imperméable. On obtient ainsi une fermeture latérale améliorée, combinant les propriétés mécaniques et adhésives de la patte d'attache.

Le document EP 529 681 décrit un article d'hygiène, du type couche-culotte, qui comprend des éléments d'attache mécaniques auto-agrippants peur la fermeture de l'article autour du corps de l'usager et des éléments d'attache adhésifs peur la fermeture de l'article après usage, replié sur lui-même. Dans une variante de réalisation, les éléments adhésifs sont constitués de pattes d'attache indépendantes des pattes d'attache supportant les éléments mécaniques du type crochets. Dans une autre variante de réalisation l'élément adhésif se trouve sous les éléments d'attache mécaniques du type crochets, ces derniers étant pelables ce qui permet de dégager la face adhésive peur son utilisation lors de la fermeture de l'article replié sur lui-même après usage. Selon une dernière variante l'élément adhésif est rapporté sur la face opposée aux éléments du type crochets, ledit élément adhésif comprenant une bande anti-adhérente pelable.

Selon le demandeur les différentes variantes proposées par le document EP 529 681 présentent toutes des inconvénients. La première variante nécessite des pattes d'attache supplémentaires à appliquer, lors de la fabrication de l'article, selon un bord de celui-ci. Cette solution rend plus complexe la fabrication et augmente le coût de production. La deuxième variante présente le risque d'une désolidarisation involontaire de l'élément du type crochet par rapport à la face adhésive lors de la traction exercée par l'utilisateur lorsqu'il ouvre le système de fermeture auto-agrippante en vue de le repositionner une nouvelle fois. La troisième variante présente les mêmes inconvénients que la première puisqu'elle nécessite de venir rapporter sur la patte d'attache latérale, d'une part une zone adhésivée et d'autre part un élément de protection anti-adhérent protégeant ladite zone pendant l'utilisation normale.

Le but que s'est fixé le demandeur est de proposer un article d'hygiène, notamment couche-culotte, qui pallie les inconvénients précités.

Il s'agit d'un article qui présente une structure qui s'apparente à celle de la troisième variante précitée du document EP 529 681.

De manière connue cet article d'hygiène comprend une feuille imperméable extérieure, une feuille perméable intérieure, un matelas absorbant disposé entre les deux dites feuilles et un système d'attache mécanique autoagrippant composé de deux pattes d'attache latérales, fixées selon les bords de la partie arrière de l'article et supportant des premiers éléments mécaniques du type crochets et au moins une bande fixée sur la partie extérieure de la partie avant de l'article et portant des seconds éléments mécaniques du type boucles, aptes à coopérer avec les premiers éléments mécaniques pour la fermeture de l'article autour du corps de l'usager. De plus chaque patte d'attache latérale comprend une bande principale dont l'extrémité proximale est fixée sur la feuille extérieure selon le bord latéral de celle-ci et dont l'extrémité distale porte les premiers éléments d'attache mécaniques.

De manière caractéristique chaque patte d'attache latérale comprend également une bande secondaire adhésive dont la face adhésivée est fixée, selon son extrémité proximale, sur la feuille perméable intérieure selon le bord latéral de celle-ci et, selon son extrémité distale, sur la bande principale à proximité immédiate des premiers éléments d'attache mécaniques. De plus la bande principale comporte une zone non adhérente, en amont de l'extrémité distale de la bande secondaire, et la bande secondaire présente un pli immédiatement en amont de la zone non adhérente et une ligne de prédécoupe dans le creux en amont dudit pli.

Dans le document EP 529 681, il y a bien également une bande secondaire adhésive dont la face adhésive est fixée, selon son extrémité proximale sur la feuille perméable intérieure, selon le bord latéral de celle-ci et dont l'extrémité distale vient s'appliquer sur la bande principale immédiatement au-delà du bord latéral de l'article. Cette bande secondaire a pour fonction de consolider la fixation de la bande principale.

De manière originale, selon l'invention cette bande secondaire a une fonction supplémentaire qui est de constituer le moyen adhésif de fermeture sur lui-même de l'article après usage. Plus précisément, l'utilisateur se saisit du pli formé sur la bande secondaire, comme d'une patte de préhension et exerce une traction sur ce pli qui entraîne corrélativement la découpe de la bande secondaire au niveau dudit pli selon la ligne de prédécoupe. En continuant de tirer sur le pli, l'utilisateur dégage la face adhésive de la bande secondaire qui se trouvait au contact de la zone non adhérente tout en rabattant cette partie de la bande secondaire par dessus les éléments mécaniques à crochets.

Il a également déjà été proposé dans la demande de brevet européen EP 324 578 un article d'hygiène jetable comportant des moyens de fermeture mécanique qui sont prévus pour la fixation et le maintien de l'article d'hygiène sur l'usager, et des moyens adhésifs, qui sont prévus pour permettre le maintien de l'article dans une configuration de mise au rebut, après par exemple que l'article ait été roulé sur lui-même. Plus particulièrement, dans une variante de réalisation illustrée sur les figures 9 et 10 de cette publication, les moyens adhésifs pour la mise au rebut de l'article d'hygiène se présentent sous la forme d'un ruban secondaire, dont l'une des faces est enduite en partie d'une couche d'un adhésif sensible à la pression. Une des extrémités de ce ruban secondaire est fixée par soudure ultra-sons directement sur le ruban de fermeture mécanique principal. La partie adhésivée de ce ruban secondaire est en outre protégée par une garniture de dégagement. Lors de la mise au rebut de l'article d'hygiène, on libère la partie adhésivée du ruban secondaire, en tirant sur l'extrémité libre de ce ruban secondaire.

Comparativement à cette variante, dans l'invention, la bande secondaire adhésivée de l'article d'hygiène est fixée par l'intermédiaire de sa face adhésivée, au niveau de ses extrémités respectivement sur le ruban d'attache principal et sur le corps de la couche. Dans l'invention, la bande secondaire adhésivée permet ainsi avantageusement de renforcer la fixation du ruban d'attache principal par rapport au corps de l'article d'hygiène, ce qui n'est pas le cas pour le ruban secondaire adhésivée de la variante précitée des figures 9 et 10 de la demande de brevet européen EP 324 578. Egalement, on évite comparativement à cette variante la mise en oeuvre d'une soudure ultrasons.

D'une manière plus générale, dans aucune des variantes de réalisation décrites dans la demande de brevet européen EP 324 578, il n'est enseigné de mettre en oeuvre comme moyen adhésif pour la mise au rebut de l'article une bande adhésive comportant un pli associé à une ligne de prédécoupe réalisée dans le creux en amont du pli. Il en résulte d'un point de vue fonctionnel que dans les articles d'hygiène décrits dans cette demande de brevet européen, les moyens décrits pour la mise au rebut ne sont pas prévus pour être séparés en deux parties, contrairement à l'invention dans laquelle le pli associé à la prédécoupe dans la bande secondaire adhésivée fait office de languette de préhension et permet par simple traction de couper en deux parties la bande secondaire selon la ligne de prédécoupe et de libérer la face adhésivée de l'une des deux parties.

Selon un mode de réalisation, la bande principale est une bande elle-même adhésivée et la zone non adhérente est constituée par un revêtement anti-adhérent qui est appliqué sur la bande principale ; un tel revêtement anti-adhérent peut être constitué par un film anti-adhérent rapporté sur la bande principale ou encore par un traitement localisé de ladite bande principale.

Selon un autre mode de réalisation, la zone non adhérente est une zone de la bande principale ne comportant pas d'adhésif.

La présente invention sera mieux comprise à la lecture de la description qui va être faite d'un exemple préféré de réalisation d'une couche-culotte munie de pattes d'attache latérales à propriété mécanique et adhésive illustré par le dessin annexé dans lequel :
- les figures 1 et 2 sont des représentations schématiques en perspective d'une partie d'un article muni d'une patte d'attache latérale avec une bande secondaire adhésive en position normale (figure 1) et en position relevée (figure 2),
- les figures 3 et 4 sont des représentations schématiques en coupe de l'article des figures 1 et 2, selon l'axe respectivement III - III et IV- IV.

La couche-culotte 1 qui est représentée partiellement sur les figures est constituée, de manière conventionnelle, par la superposition d'une feuille extérieure imperméable 2, d'une feuille intérieure perméable 3 et d'un matelas absorbant 4. Les feuilles extérieure 2 et intérieure 3 sont solidarisées l'une à l'autre le long des bords périphériques de la couche, tandis que le matelas absorbant, de forme généralement rectangulaire, occupe la partie centrale de celle-ci. La fermeture de la couche-culotte 1 autour du corps de l'usager est réalisée grâce à un système d'attache mécanique, du type auto-agrippant, qui comprend d'une part deux pattes d'attache latérales équipant les bords latéraux de la partie arrière de la couche-culotte 1 et d'autre part au moins une bande frontale disposée sur la face extérieure de la partie avant de la couche-culotte 1. Les pattes latérales sont équipées d'éléments d'attache mécaniques du type crochets, tandis que la bande frontale est équipée d'éléments mécaniques du type boucles, les crochets et les boucles étant aptes à coopérer les uns avec les autres pour réaliser l'accrochage et donc le blocage en position desdites pattes d'attache sur ladite bande frontale.

De manière caractéristique selon l'invention, la couche-culotte 1 est équipée de pattes d'attache latérales 5 qui comportent non seulement des éléments mécaniques du type crochets 6 mais également un moyen adhésif relevable, comme cela va être décrit ci-après. Cette patte d'attache latérale 5 est constituée d'une bande principale 7 qui est de préférence une bande adhésive dont une extrémité 7a dite extrémité proximale est fixée le long du bord latéral 1a de la couche 1, plus précisément sur la feuille extérieure imperméable 2.

Dans le présent texte, l'adjectif proximal est utilisé en référence au matelas absorbant 4. Ainsi l'extrémité proximale 7a de la bande principale 7 est l'extrémité qui est la plus proche du matelas absorbant 4. Par opposition est dénommée distale l'extrémité 7b de la bande principale 7 la plus éloignée du matelas absorbant 4. C'est vers cette extrémité distale 7b que sont disposés les éléments d'attache mécaniques du types crochets 6, sur la face adhésivée 7' de la bande principale 7.

Dans le présent texte on utilise également les termes amont et aval en référence au matelas absorbant 4. Est considéré comme étant en amont ce qui est plus proche du matelas absorbant et en aval ce qui est plus éloigné du matelas absorbant 4.

La patte latérale d'attache 5 comporte également une bande secondaire adhésive 8 dont l'extrémité proximale 8a est fixée selon le bord latéral 1a de la couche-culotte 1, plus précisément sur la feuille intérieure perméable 3 et dont l'extrémité distale 8b est fixée sur la bande principale 5 à proximité immédiate des éléments du type crochets 6, en amont de ceux-ci.

En amont de l'extrémité distale 8b de la bande secondaire 8 est prévu, sur la face adhésivée 7' de la bande principale 7, un revêtement anti-adhérent 9, constitué d'un film anti-adhérent rapporté sur ladite face adhésivée 7', qui occupe toute la largeur 1 de la patte latérale 5 et qui s'étend sur une distance d déterminée.

La bande secondaire 8 depuis son extrémité proximale 8a s'applique successivement sur la feuille perméable intérieure 3 puis sur la face adhésivée 7' de la bande principale 7, sur le revêtement anti-adhérent 9 et enfin, à son extrémité distale 8b, sur la face adhésivée 7' de la bande principale 7. De plus, en amont du revêtement anti-adhérent 9, la bande secondaire 8 forme un pli 10 qui s'étend transversalement par rapport à la patte d'attache latérale 5, le sommet 11 du pli 10 étant parallèle au bord latéral 1a de la couche-culotte 1. Dans le creux 12 du pli 10 qui est le plus en amont, la bande secondaire 8 comporte une prédécoupe 13, transversalement par rapport à la patte d'attache latérale 5, se présentant sous forme de traits 14 discontinus.

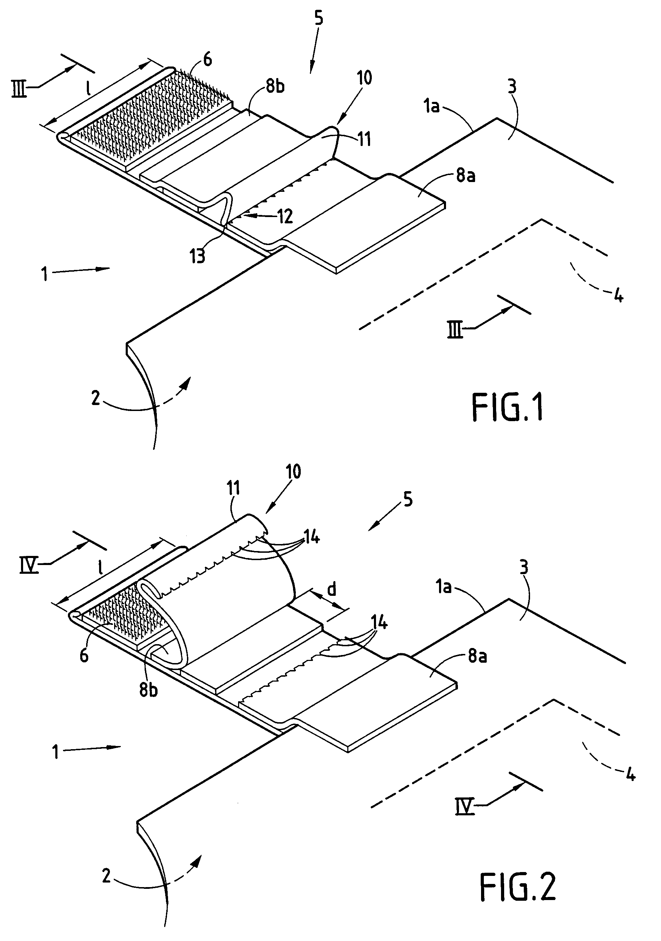

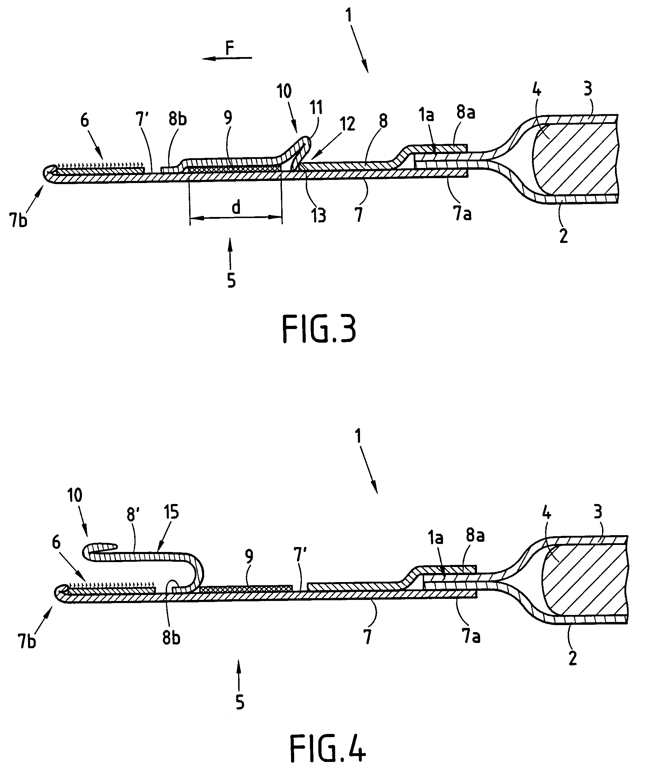

Lors de l'utilisation normale de la patte d'attache latérale 5 pour la fermeture de la couche-culotte 1 autour du corps de l'usager, la patte d'attache 5 présente la configuration qui est illustrée aux figures 1 et 3 ; seuls les éléments d'attache mécaniques du type crochets 6 sont mis en oeuvre à l'occasion de cette utilisation.

Par contre lorsque la couche-culotte 1 est destinée à être jetée, l'utilisateur l'enroule sur elle-même et utilise la patte d'attache latérale 5 pour réaliser la fixation adhésive de la dite patte 5 sur un élément quelconque de la couche-culotte 1 et, en particulier sur la feuille extérieure imperméable 2. Pour ce faire, l'utilisateur se saisit du pli 10, qui fait office de languette de préhension, exerce une traction dans le sens de la flèche F, ce qui a pour effet de couper en deux parties la bande secondaire 8, selon la ligne de prédécoupe 13 au niveau du creux 12 du pli 10, à savoir une première partie amont, comprenant l'extrémité proximale 8a qui reste collée d'une part sur la bande principale 7 et d'autre part sur le bord latéral 2 de la feuille intérieure perméable 3 et une seconde partie aval 15 qui n'est collée sur la face adhésivée 7' de la bande principale 7 que selon son extrémité distale 8b. En continuant à exercer la traction, selon la flèche F, l'utilisateur peut ainsi faire pivoter la partie aval 15 de la bande secondaire 8 autour de l'extrémité distale 8b fixe , ce qui a pour effet d'une part de dégager la face adhésivée 8' de la bande secondaire 8 au niveau de ladite partie aval 15 et d'autre part de recouvrir les éléments d'attache mécaniques du type crochets 6.

Lorsque l'utilisateur met en oeuvre la face adhésivée 8' de la seconde partie aval 15, comme d'une patte d'attache adhésive, pour fermer la couche-culotte 1, après que celle-ci ait été repliée sur elle-même après usage, les éléments mécaniques du type crochets viennent en contact avec la face extérieure non adhésive de la partie aval 15 et sont donc inactifs.

On comprend que la structure de la patte d'attache latérale 5 est particulièrement simple et de fabrication facilitée puisqu'elle est constituée uniquement de quatre éléments distincts, à savoir la bande principale 7, la bande secondaire 8, le revêtement anti-adhérent 9 et les éléments du type crochets 6.

Il serait encore possible de simplifier cette structure en supprimant le revêtement anti-adhérent 9 et en utilisant une bande principale 5 ayant localement une zone non adhésivée sur la même distance d, de sorte que cette zone anti-adhérente puisse jouer le même rôle que ce qui a été décrit ci-dessus pour le revêtement anti-adhérent 9, à savoir de préserver la face adhésivée 8' de la partie aval 15 de la bande secondaire 8.

## Revendications

1. Article d'hygiène (1), notamment couche-culotte, comprenant une feuille imperméable extérieure (2), une feuille perméable intérieure (3), un matelas absorbant (4) disposé entre les deux dites feuilles (2, 3) et un système d'attache mécanique auto-agrippant composé de deux pattes d'attache latérales (5), fixées selon les bords (1a) de la partie arrière de l'article et supportant des premiers éléments mécaniques du type crochets (6) et au moins une bande fixée sur la partie extérieure de la partie avant de l'article et portant des seconds éléments mécaniques du type boucles, aptes à coopérer avec les premiers éléments mécaniques (6) pour la fermeture de l'article autour du corps de l'usager, chaque patte d'attache latérale (5) comprenant une bande principale (7) dont l'extrémité proximale (7a) est fixée sur la feuille extérieure (2) selon le bord latéral de celle-ci et dont l'extrémité distale (7b) porte les premiers éléments d'attache mécaniques (6) caractérisé en ce que chaque patte d'attache latérale (7) comprend également une bande secondaire adhésive (8) dont la face adhésivée (8) est fixée, selon son extrémité proximale (8a), sur la feuille perméable intérieure (3) selon le bord latéral de celle-ci et, selon son extrémité distale (8b), sur la bande principale (7) à proximité immédiate des premiers éléments d'attache mécaniques (6), en ce que la bande principale (7) comporte une zone non adhérente (9), en amont de l'extrémité distale (8b) de la bande secondaire (8), et en ce que la bande secondaire (8) présente un pli (10) immédiatement en amont de la zone non adhérente (9) et une ligne de prédécoupe (13) dans le creux (12) en amont dudit pli (10).

2. Article selon la revendication 1 caractérisé en ce que la bande principale (7) est une bande elle-même adhésivée et la zone non adhérente est constituée par un revêtement anti-adhérent (9) qui est appliqué sur la bande principale (7).

3. Article selon la revendication 1 caractérisé en ce que la zone non adhérente est une zone de la bande principale (7) ne comportant pas d'adhésif.
